# EUROPEAN PATENT SPECIFICATION

(11) **EP 1 317 306 B1**
(45) Date of publication and mention of the grant of the patent: **20.04.2005**
(21) Application number: 01970743.9
(22) Date of filing: 10.09.2001
(51) Int. Cl.: A61N 1/30

(54) **TRANSDERMAL ELECTROTRANSPORT DEVICE AND METHOD FOR MANUFACTURING SAME**
VORRICHTUNG ZUR TRANSDERMALEN VERABREICHUNG VON MEDIKAMENTEN UND ENTSPRECHENDES HERSTELLUNGSVERFAHREN
DISPOSITIF D'ELECTROTRANSPORT TRANSDERMIQUE ET PROCEDE DE FABRICATION CORRESPONDANT

(30) Priority: 11.09.2000 US 231898 P
(43) Date of publication of application: 11.06.2003
(73) Proprietor: Alza Corporation, Mountain View, CA 94039-7210 (US)
(72) Inventor: KLEINER, Lothar, W., Los Altos, CA 94024 (US); YOUNG, Wendy, A., San Jose, CA 95129 (US)
(74) Representative: Mercer, Christopher Paul
(86) International application number: PCT/US2001/028258
(87) International publication number: WO 2002/022204

(56) References cited:
- EP-A- 1 016 433
- WO-A-00/25858
- WO-A-96/34597
- US-A- 5 006 108
- US-A- 6 071 508

## Description

### Technical Field

The invention relates generally to a device for electrotransport delivery of a therapeutic agent, and more particularly, to an electrotransport device having a housing made from an ethylene-octene copolymer, and a method for manufacturing the same.

### Background Art

The transdermal delivery of drugs, by diffusion through the skin, offers improvements over more traditional delivery methods, such as subcutaneous injections and oral delivery. Transdermal drug delivery avoids the hepatic first pass effect encountered with oral drug delivery. Transdermal drug delivery also eliminates patient discomfort associated with subcutaneous injections. In addition, transdermal delivery can provide more uniform concentrations of drug in the bloodstream of the patient over time due to the extended controlled delivery profiles of certain types of transdermal delivery devices. The term "transdermal" as used herein broadly encompasses the delivery of an agent through a body surface, such as the skin, mucosa, or nails of an animal.

The skin functions as the primary barrier to the transdermal penetration of materials into the body and represents the body's major resistance to the transdermal delivery of therapeutic agents such as drugs. To date, efforts have been focused on reducing the physical resistance or enhancing the permeability of the skin for the delivery of drugs by passive diffusion. Various methods for increasing the rate of transdermal drug flux have been attempted, most notably using chemical flux enhancers.

Other approaches to increase the rates of transdermal drug delivery include use of alternative energy sources such as electrical energy and ultrasonic energy. Electrically assisted transdermal delivery is also referred to as electrotransport. The term "electrotransport" as used herein refers generally to the delivery, extraction, or sampling of an agent (e.g., a drug, a body analyte, or the like) through a membrane, such as skin, mucous membrane, or nails. The delivery is induced or aided by application of an electrical potential. For example, a beneficial therapeutic agent may be introduced into the systemic circulation of a human body by electrotransport delivery through the skin. A widely used electrotransport process, electromigration (also called iontophoresis), involves the electrically induced transport of charged ions. Another type of electrotransport, electroosmosis, involves the flow of a liquid, which liquid contains the agent to be delivered, under the influence of an electric field. Still another type of electrotransport process, electroporation, involves the formation of transiently-existing pores in a biological membrane by the application of an electric field. An agent can be delivered through the pores either passively (i.e., without electrical assistance) or actively (i.e., under the influence of an electric potential). However, in any given electrotransport process, more than one of these processes, including at least some "passive" diffusion, may be occurring simultaneously to a certain extent. Accordingly, the term "electrotransport", as used herein, should be given its broadest possible interpretation so that it includes the electrically induced or enhanced transport of at least one agent, which may be charged, uncharged, or a mixture thereof, whatever the specific mechanism or mechanisms by which the agent actually is transported.

The term "agent" is intended to have its broadest interpretation and is used to include any therapeutic agent or drug, as well as any body analyte, such as glucose. The terms "drug" and "therapeutic agent" are used interchangeably to refer to any therapeutically active substance that is delivered to a living organism to produce a desired, usually beneficial, effect. This includes therapeutic agents in all the major therapeutic areas including, but not limited to: anti-infectives such as antibiotics and antiviral agents; analgesics, including fentanyl, sufentanil, buprenorphine and analgesic combinations; anesthetics; anorexics; antiarthritics; antiasthmatic agents such as terbutaline; anticonvulsants; antidepressants; antidiabetic agents; antidiarrheals; antihistamines; anti-inflammatory agents; antimigraine preparations; antimotion sickness preparations such as scopolamine and ondansetron; antinauseants; antineoplastics; antiparkinsonism drugs; antipruritics; antipsychotics; antipyretics; antispasmodics, including gastrointestinal and urinary; anticholinergics; sympathomimetrics; xanthine derivatives; cardiovascular preparations, including calcium channel blockers such as nifedipine; beta blockers; beta-agonists such as dobutamine and ritodrine; antiarrythmics; antihypertensives such as atenolol; ACE inhibitors such as ranitidine; diuretics; vasodilators, including general, coronary, peripheral, and cerebral; central nervous system stimulants; cough and cold preparations; decongestants; diagnostics; hormones such as parathyroid hormone; hypnotics; immunosuppressants; muscle relaxants; parasympatholytics; parasympathomimetrics; prostaglandins; proteins; peptides; psychostimulants; sedatives; and tranquilizers.

Of particular interest in transdermal delivery is the delivery of analgesic drugs for the management of moderate to severe pain. Control of the rate and duration of drug delivery is particularly important for transdermal delivery of analgesic drugs to avoid the potential risk of overdose and the discomfort of an insufficient dosage. One class of analgesics that has found application in a transdermal delivery route is the synthetic opiates, a group of 4-aniline piperidines. The synthetic opiates, e.g., fentanyl and certain of its derivatives such as sufentanil, are particularly well-suited for transdermal administration. These synthetic opiates are characterized by their rapid onset of analgesia, high potency, and short duration of action. They are estimated to be 80 and 800 times, respectively, more potent than morphine. These drugs are weak bases, i.e., amines, whose major fraction is cationic in acidic media.

Electrotransport devices use at least two electrodes that are in electrical contact with some portion of the skin, nails, mucous membrane, or other surface of the body. One electrode, commonly called the "donor" electrode, is the electrode from which the agent is delivered into the body. The other electrode, typically termed the "counter" electrode, serves to close the electrical circuit through the body. For example, if the agent to be delivered is positively charged, i.e., a cation, then the anode is the donor electrode, while the cathode is the counter electrode which serves to complete the circuit. Alternatively, if an agent is negatively charged, i.e., an anion, the cathode is the donor electrode and the anode is the counter electrode. Additionally, both the anode and cathode may be considered donor electrodes if both anionic and cationic agent ions, or if uncharged dissolved agents, are to be delivered.

Furthermore, electrotransport delivery systems generally require at least one reservoir or source of the agent to be delivered to the body. Examples of such donor reservoirs include a pouch or cavity, a porous sponge or pad, and a hydrophilic polymer or a gel matrix. Such donor reservoirs are electrically connected to, and positioned between, the anode or cathode and the body surface, to provide a fixed or renewable source of one or more agents or drugs. Electrotransport devices also have an electrical power source such as one or more batteries. Typically at any one time, one pole of the power source is electrically connected to the donor electrode, while the opposite pole is electrically connected to the counter electrode. Since it has been shown that the rate of electrotransport drug delivery is approximately proportional to the electric current applied by the device, many electrotransport devices typically have an electrical controller that controls the voltage and/or current applied through the electrodes, thereby regulating the rate of drug delivery. These control circuits use a variety of electrical components to control the amplitude, polarity, timing, waveform shape, etc. of the electric current and/or voltage supplied by the power source. See, for example, McNichols et al., U.S. Patent 5,047,007.

To date, commercial transdermal electrotransport drug delivery devices (e.g., the Phoresor, sold by lomed, Inc. of Salt Lake City, UT; the Dupel lontophoresis System sold by Empi, Inc. of St. Paul, MN; the Webster Sweat Inducer, model 3600, sold by Wescor, Inc. of Logan, UT) have generally utilized a desk-top electrical power supply unit and a pair of skin contacting electrodes. The donor electrode contains a drug solution while the counter electrode contains a solution of a biocompatible electrolyte salt. The power supply unit has electrical controls for adjusting the amount of electrical current applied through the electrodes. The "satellite" electrodes are connected to the electrical power supply unit by long (e.g., 1-2 meters) electrically conductive wires or cables. The wire connections are subject to disconnection and limit the patient's movement and mobility. Wires between electrodes and controls may also be annoying or uncomfortable to the patient. Other examples of desk-top electrical power supply units which use "satellite" electrode assemblies are disclosed in Jacobsen et al., U.S. Patent 4,141,359 (see Figures 3 and 4); LaPrade, U.S. Patent 5,006,108 (see Figure 9); and Maurer et al., U.S. Patent 5,254,081.

More recently, electrotransport delivery devices have become much smaller, particularly with the development of miniaturized electrical circuits (e.g., integrated circuits) and more powerful light weight batteries (e.g., lithium batteries). The advent of inexpensive miniaturized electronic circuitry and compact, high-energy batteries has meant that the entire device can be made small enough to be unobtrusively worn on the skin of the patient, under clothing. This allows the patient to remain fully ambulatory and able to perform all normal activities, even during periods when the electrotransport device is actively delivering drug. Such small self-contained electrotransport delivery devices are disclosed for example in Tapper, U.S. Patent 5,224,927; Sibalis, et al., U.S. Patent 5,224,928; and Haynes et al., U.S. Patent 5,246,418.

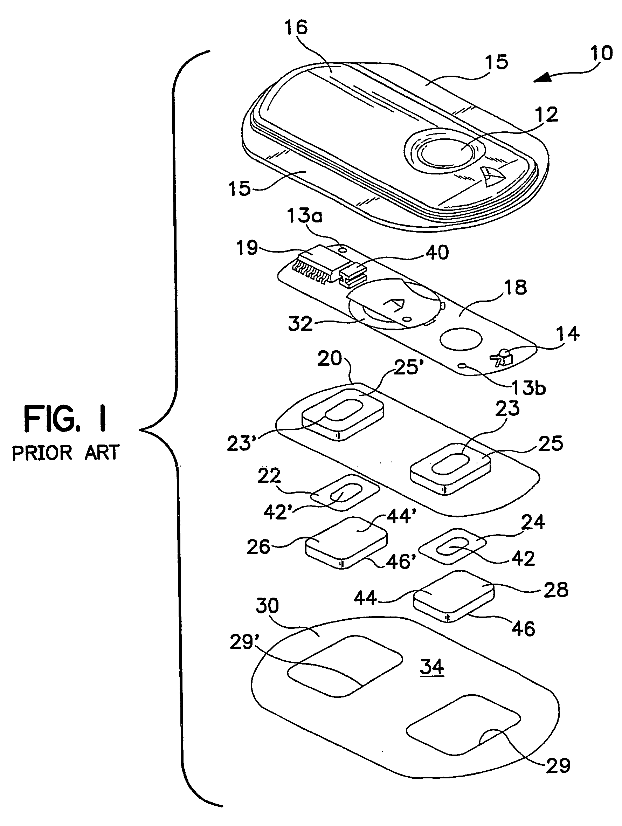

Reference is now made to FIG. 1 which depicts an exploded view of an exemplary electrotransport device 10 having an activation switch in the form of a push button switch 12 and a display in the form of a light emitting diode (LED) 14. Device 10 comprises an upper housing 16, a circuit board assembly 18, a lower housing 20, anode electrode 22, cathode electrode 24, anode reservoir 26, cathode reservoir 28 and skin-compatible adhesive 30. Upper housing 16 has lateral wings 15 which assist in holding device 10 on a patient's skin. Upper housing 16 is generally composed of rubber or other elastomeric material, such as an ethylene vinyl acetate copolymer having 28% vinyl acetate (EVA-28). Lower housing 20 is typically composed of a plastic or elastomeric sheet material (such as, e.g., polyethylene terephthalate glycol (PETG) or polyethylene) which can be easily molded or thermoformed to form depressions and cut to form openings therein. Printed circuit board assembly 18 comprises an integrated circuit 19 coupled to discrete electrical components 40 and battery 32. Circuit board assembly 18 is attached to housing 16 by posts (not shown in FIG. 1) passing through openings 13a and 13b, the ends of the posts being heated/melted in order to heat stake the circuit board assembly 18 to the housing 16. Lower housing 20 is attached to the upper housing 16 by means of adhesive 30, the upper surface 34 of adhesive 30 being adhered to both lower housing 20 and upper housing 16 including the bottom surfaces of wings 15.

On the underside of circuit board assembly 18 is a battery 32, which may be a button cell battery, such as a lithium cell. The circuit outputs of the circuit board assembly 18 make electrical contact with the electrodes 24 and 22 through openings 23,23' in the depressions 25,25' formed in lower housing, by means of electrically conductive adhesive strips 42,42'. Electrodes 22 and 24, in turn, are in direct mechanical and electrical contact with the top sides 44',44 of drug reservoir 26 and electrolyte reservoir 28. The bottom sides 46',46 of reservoirs 26,28 contact the patient's skin through the openings 29',29 in adhesive 30. Upon depression of push button switch 12, the electronic circuitry on circuit board assembly 18 delivers a predetermined DC current to the electrodes/reservoirs 22,26 and 24,28 for a delivery interval of predetermined length.

Electrotransport delivery devices are prepared, shipped and stored (or stored, shipped and stored), prescribed and then used. As a result, the devices must have components that have extended shelf lives that, in some instances, must comply with regulatory requirements. For instance, the U.S. Food and Drug Administration has shelf life requirements of from six to eighteen months for some materials. One complicating factor in achieving an extended shelf life is the dimensional stability of EVA-28 when exposed to elevated temperatures. In order to achieve satisfactory dimensional stability of the device housing when it is manufactured from EVA-28, for example, the molding conditions must be carefully optimized, thus limiting the processing window. Otherwise warpage as well as unacceptable dimensional changes will occur at temperatures as low as 40°C. If the device housing should encounter excessive heat during storage or shipping, however, these same undesirable dimensional changes can occur. Further, electrotransport delivery devices typically contain electronic components (e.g., integrated circuits), conductive circuit traces and electrical connections therebetween which can corrode or otherwise be degraded by water or water vapor. Unfortunately, devices such as device 10 shown in FIG. 1 have hydratable or hydrated reservoirs 26, 28. Thus, humidity or moisture from the hydrated reservoirs can permeate through the device housing during manufacturing and storage, which can thus cause corrosion of the electronic components within the device, thereby reducing the shelf life of the device. Finally, it has also been found that upon long term storage of devices having housings that are made from ethylene vinyl acetate copolymers, such as EVA-28, the housing can release a low level residual acetic acid vapor. This acetic acid off-gassing will react with metals to form an acetate, e.g., Ni acetate, which can cause corrosion problems in the electronic components housed within the device.

In view of the above, a strong need therefore exists for a polymeric material that can be fabricated into an electrotransport housing, which has increased dimensional stability, and thus improved heat resistance, which demonstrates improved processing (e.g., molding) characteristics, which is chemically inert, and which has lower moisture vapor transmission properties and no off-gassing (such as acetic acid) so as to reduce the likelihood of component corrosion.

### Disclosure of the Invention

The present invention overcomes these disadvantages and provides an electrotransport device having an increased shelf life and a method of making same. The device of the present invention includes a housing enclosing the electronic components. The device further includes at least one hydrated reservoir, and more typically at least two hydrated reservoirs. According to the present invention, at least a portion of the housing of the device is comprised of an ethylene-octene copolymer. In a preferred mode of the invention, the housing includes an upper, exterior housing portion and a lower housing portion, and at least the upper housing portion is comprised of an ethylene-octene copolymer. The use of ethylene-octene for at least the upper or exterior housing of the electrotransport device provides improved molding properties and an increased processing window when compared with the prior use of EVA-28, good flexibility and the specified hardness in the final molded product, improved dimensional stability so as to retain the specified dimensions of the molded part, a lower moisture vapor transmission rate which reduces the risk of corroding the electronic components, and no acetic acid off-gassing to corrode the electronic components (or the injection molding tooling).

Other advantages and a fuller appreciation of specific adaptations, compositional variations, and physical attributes of the present invention can be learned from an examination of the following drawings, detailed description, examples, and appended claims.

### Brief Description of the Drawings

These, and other, objects, features and advantages of the present invention will become more readily apparent to those skilled in the art upon reading the following detailed description, in conjunction with the appended drawings, in which:

FIG. 1 is an exploded perspective view of a known electrotransport drug delivery device; and

FIG. 2 is a perspective exploded view of an electrotransport drug delivery device in accordance with the present invention.

### Modes for Carrying Out the Invention

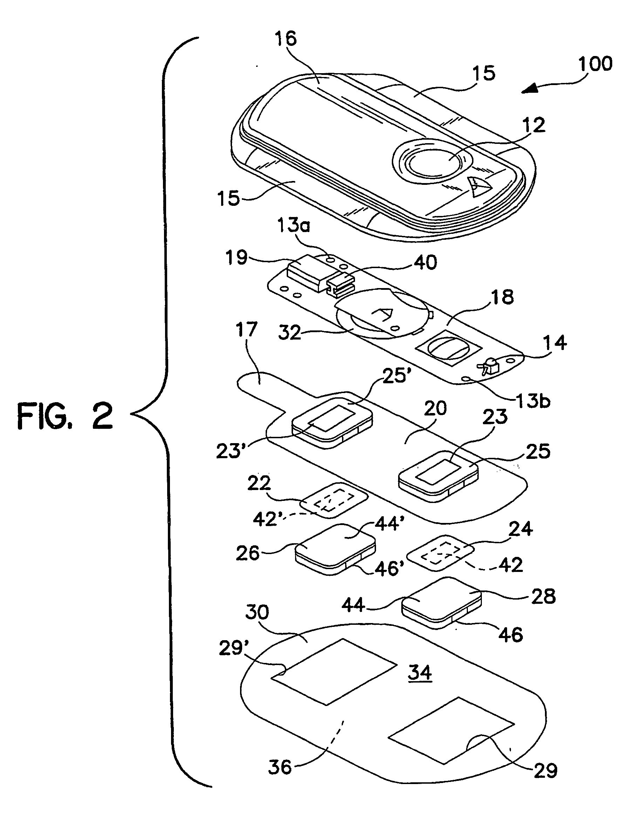

An example of an electrotransport delivery device of the present invention is illustrated in FIG. 2. With reference to FIG. 2, there is shown a perspective view of an electrotransport device 100 having an optional activation switch in the form of a push button switch 12 and an optional light emitting diode (LED) 14 which turns on when the device 10 is in operation.

Device 100 comprises an upper or exterior housing 16, a circuit board assembly 18, a lower housing 20, anode electrode 22, cathode electrode 24, anode reservoir 26, cathode reservoir 28 and skin-compatible adhesive 30. Upper housing 16 has lateral wings 15 which assist in holding device 100 on a patient's skin. Lower housing 20 has a projecting pull tab 17 which assists in removing reservoirs 26 and 28 from the electronic components of device 100 following use on a patient (at least one of the reservoirs 26 and 28 may contain residual drug, for example a narcotic drug or other controlled substance, which must be separately disposed for safety reasons). Printed circuit board assembly 18 comprises an integrated circuit 19 coupled to discrete components 40 and battery 32. Circuit board assembly 18 is attached to housing 16 by posts (not shown in FIG. 2) passing through openings 13a and 13b. The ends of the posts are heated/melted in order to heat stake the circuit board assembly 18 to the housing 16. Lower housing 20 is attached to the upper housing 16 by means of adhesive 30, the upper surface 34 of adhesive 30 being adhered to both lower housing 20 and upper housing 16 including the bottom surfaces of wings 15, but not the bottom surface of pull tab 17.

Shown (partially) on the underside of circuit board assembly 18 is a button cell battery 32. Other types of batteries may also be employed to power device 100. The device 100 is generally comprised of battery 32, electronic circuitry 19,40, electrodes 22,24, and drug/chemical reservoirs 26,28, all of which are integrated into a self-contained unit. The outputs (not shown in FIG. 2) of the circuit board assembly 18 make electrical contact with the electrodes 24 and 22 through openings 23,23' in the depressions 25,25' formed in lower housing 20, by means of electrically conductive adhesive strips 42,42'. Electrodes 22 and 24, in turn, are in direct mechanical and electrical contact with the top sides 44',44 of reservoirs 26 and 28. The bottom sides 46',46 of reservoirs 26,28 contact the patient's skin through the openings 29',29 in adhesive 30.

Upon depression of push button switch 12, the electronic circuitry on circuit board assembly 18 delivers a predetermined DC current to the electrodes/reservoirs 22,26 and 24,28 for a delivery interval of predetermined length. Preferably, the device transmits to the user a visual and/or audible confirmation of the onset of the drug delivery by means of LED 14 becoming lit and/or an audible sound signal from, e.g., a "beeper". Drug is thereby delivered from one (or both) of reservoirs 26, 28 and through the patient's skin by electrotransport.

Anodic electrode 22 is preferably comprised of silver and cathodic electrode 24 is preferably comprised of silver chloride. Both reservoirs 26 and 28 are preferably comprised of polymer hydrogel materials. Electrodes 22, 24 and reservoirs 26, 28 are retained by lower housing 20. In one aspect of the present invention, one of reservoirs 26, 28 is the "donor" reservoir and contains the agent (e.g., a drug) to be delivered and the other reservoir typically contains a biocompatible electrolyte. In a further aspect of the present invention, one of reservoirs is an acceptor or analysis reservoir and receives an extracted body analyte, such as glucose, from the body through the use of reverse electrotransport.

The push button switch 12, the electronic circuitry on circuit board assembly 18 and the battery 32 are adhesively "sealed" between upper housing 16 and lower housing 20. Upper or exterior housing 16 is preferably composed of an ethylene-octene copolymer material, as discussed in greater detail below. Lower housing 20 may be composed of a plastic or elastomeric sheet material (e.g., PETG or polyethylene) which can be easily molded or thermoformed to form depressions 25, 25' and cut to form openings 23, 23'. It is also within the scope of the present invention and most preferable, however, to also form lower housing 20 from an ethylene-octene copolymer since the lower moisture vapor transmission rate thereof would be beneficial in separating the hydrogel reservoirs 26, 28 from the electronics 18. The assembled device 100 is preferably water resistant (i.e., splash proof) and is most preferably waterproof. The system has a low profile that easily conforms to the body thereby allowing freedom of movement at, and around, the wearing site. The reservoirs 26, 28 are located on the skin-contacting side of the device 100 and are sufficiently separated to prevent accidental electrical shorting during normal handling and use.

The device 100 adheres to the patient's body surface (e.g., skin) by means of a peripheral adhesive 30 which has upper side 34 and body-contacting side 36. The adhesive side 36 has adhesive properties which assures that the device 100 remains in place on the body during normal user activity, and yet permits reasonable removal after the predetermined (e.g., 24-hour) wear period. Upper adhesive side 34 adheres to lower housing 20 and retains the electrodes and drug reservoirs within housing depression 25, 25' as well as retains lower housing 20 attached to upper housing 16. The push button switch 12 is conveniently located on the top side of device 100 and is easily actuated through clothing. A double press of the push button switch 12 within a short time period, e.g., three seconds, is preferably used to activate the device for delivery of drug, thereby minimizing the likelihood of inadvertent actuation of the device 100.

As mentioned above, the preferred material for the housing of device 100 of the present invention is an ethylene-octene copolymer, and most preferably those available under the tradename ENGAGE® from Dupont Dow Elastomers, Wilmington, DE. ENGAGE® is available in various grades, as shown in Table I below, the grades being distinguishable based primarily upon the percentage of octene that is present in the material and the resulting effect on the material properties.

**TABLE I**

| | | | | | | | ASTM D-638M-90, 50 mm/min | |
|---|---|---|---|---|---|---|---|---|
| Grade of Engage | % octene | Density (g/cm³) ASTM D-792 | Mooney Viscosity ML 1+4 at 121°C ASTM D-1646 | Melt Flow index (dg/min) ASTM D-1238 | Hardness shore: A ASTM D-2240 | DSC melting peak (°C) Rate 10°C/min | Ultimate Tensile strength (MPa) | Ultimate elongation (%) |
| 8180 | 28 | 0.863 | 35 | 0.5 | 66 | 49 | 10.1 | 800 |
| 8150 | 25 | 0.868 | 35 | 0.5 | 75 | 55 | 15.4 | 750 |
| 8100 | 24 | 0.870 | 23 | 1.0 | 75 | 60 | 16.3 | 750 |
| 8840 | 25 | 0.868 | 35 | 0.5 | 75 | 55 | 15.4 | 750 |
| 8200 | 24 | 0.870 | 8 | 5.0 | 75 | 60 | 9.3 | >1000 |
| 8400 | 24 | 0.870 | 1.5 | 30 | 72 | 60 | 4.1 | >1000 |
| 8452 | 22 | 0.875 | 11 | 3.0 | 79 | 67 | 17.5 | >1000 |
| 8411 | 20 | 0.880 | 3 | 18 | 76 | 78 | 10.6 | 1000 |
| 8003 | 18 | 0.885 | 22 | 1.0 | 86 | 76 | 30.3 | 700 |
| 8585 | 18 | 0.885 | 12 | 2.5 | 86 | 76 | 25.5 | 800 |
| 8440 | 14 | 0.897 | 16 | 1.6 | 92 | 95 | 32.6 | 710 |
| 8480 | 12 | 0.902 | 18 | 1.0 | 95 | 100 | 35.3 | 750 |
| 8450 | 12 | 0.902 | 10 | 3.0 | 94 | 98 | 30.7 | 750 |
| 8550 | 13.8 | 0.902 | 7 | 4.3 | 94 | 98 | 30.4 | 800 |
| 8402 | 13.5 | 0.902 | 1.5 | 30 | 94 | 100 | 14.1 | 940 |
| 8540 | 9.5 | 0.908 | 18 | 1.0 | 94 | 103 | 33.8 | 700 |
| 8445 | 9.5 | 0.910 | 8 | 3.5 | 94 | 103 | 27.9 | 750 |
| 8403 | 9.5 | 0.913 | 1.5 | 30 | 96 | 107 | 13.7 | 700 |

When selecting a material for the housing of device 100, the hardness/flexibility of the material for the housing must be sufficiently rigid so as to protect the underlying electronic components within the device, yet flexible enough to conform to the contours of the body when worn by the patient. The hardness/flexibility of ethylene-octene copolymers is determined generally by the octene concentration within the material. A preferred range of the octene content in the ethylene-octene copolymer used in the present invention is from about 5% to about 30%. In order to obtain an acceptable hardness/flexibility, one that is similar to that obtained from EVA-28, an octene percentage between about 9.5% and about 24% is preferred. Thus, the preferred grades of ENGAGE® for use in the housing of device 100 include ENGAGE® 8400, 8411, 8401, 8402 and 8403, with ENGAGE® 8411 being the most preferred. As shown in Table I above, the Shore A hardness value for ENGAGE® 8411 is 76, which is comparable to the Shore A hardness value for EVA-28 of 78.

ENGAGE® copolymers also provide an increased temperature window for processing when compared with prior elastomers, such as EVA-28. ENGAGE® is a thermally and dimensionally stable product and has a processing window for injection molding ranging from approximately 175°C to approximately 290°C for all grades of the material. ENGAGE® copolymers also have superior processing characteristics relating to the melt flow and melt stability. Thus, when compared to the injection molding processing window for EVA-28, approximately 160°C to approximately 200°C, it is apparent that easier processing and greater production will be achieved without any off-gassing of acetic acid which is corrosive to the mold and manufacturing tooling and the electronic components enclosed therein.

The greater thermal and dimensional stability of ENGAGE® copolymers also reduces the likelihood of warpage or shrinkage of the housing following injection molding, and also reduces the likelihood of detrimental dimensional changes occurring during long term storage of the device 100. The following table, Table II, demonstrates the results of a test wherein molded parts made from ENGAGE® 8401 and EVA-28 were stored at 40, 45, 50 and 55°C for two hours and then evaluated for dimensional changes.

**TABLE II**

| Temperature (°C) | EVA-28 (% change) | ENGAGE® 8401 (% change) |
|---|---|---|
| 40 | -1.84 | -0.76 |
| 45 | -2.21 | -0.90 |
| 50 | -2.69 | -0.97 |
| 55 | -4.19 | -1.25 |

As shown, as the temperature was increased from 40°C to 55°C, the molded part dimensional changes increased for both materials. However, ENGAGE® 8401 parts demonstrated less shrinkage than the EVA-28 parts. The greater thermal stability of ENGAGE® copolymers makes it an ideal material for molding the housing of the electrotransport device 100, and thereby increasing the shelf life of the device.

The moisture vapor transmission rate (MVTR) of a material generally defines the quantity of moisture that a material will allow to permeate therethrough. Since the device 100 of the present invention contains electronic components that can be adversely effected by moisture or humidity which permeates through the housing of the device, it is desirable to have as low an MVTR as possible. The table below, Table III, illustrates the MVTR values for various grades of ENGAGE® copolymers.

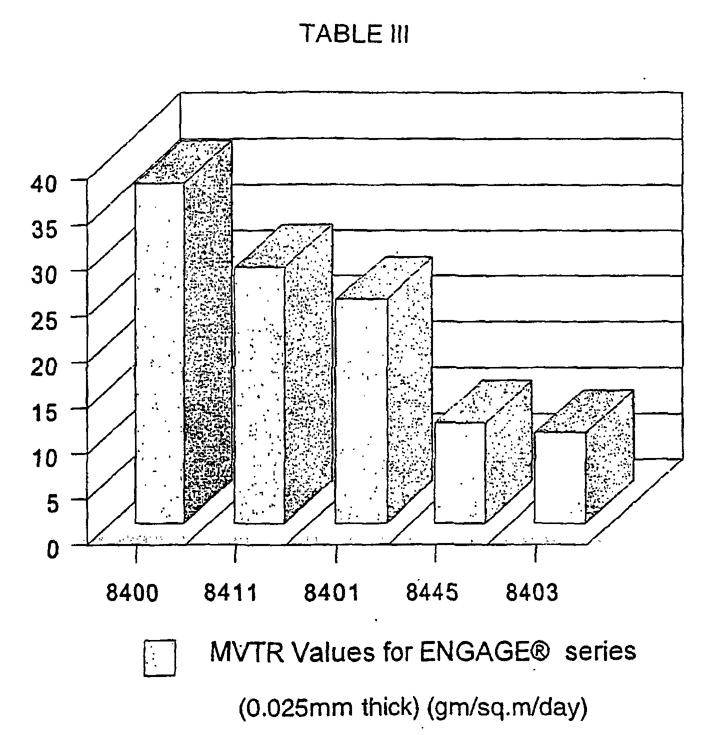

When compared with the corresponding MVTR value for EVA-28, 2188 gm/m²/day, the improved water resistance which can be obtained for device 100 is apparent. The resultant device 100 of the present invention is splash proof, which is beneficial to patients during their normal day-to-day activities (e.g., bathing).

Finally, ENGAGE® copolymers do not contain acetate.
Accordingly, there is no off-gassing of acetic acid which can lead to corrosion of the electronic components and corrosion of the tooling and molds during injection molding. The absence of acetic acid off-gassing and subsequent corrosion of the enclosed electronics during manufacture and storage also thus increases the shelf life of the product.

The device 100 of the present invention is preferably manufactured by injection molding the upper housing 16 from an ethylene-octene copolymer, preferably an ENGAGE® copolymer, most preferably ENGAGE® 8411 is used. The lower housing 20 can be injection molded or thermoformed from an elastomeric sheet, such as polyethylene or PETG. It is within the scope of the present invention however, to also provide an ethylene-octene copolymer, preferably an ENGAGE® copolymer, having sufficient properties for thermoforming so as to allow lower housing 20 to be made therefrom or for injection molding the lower housing 20 from an ENGAGE® copolymer similar to the grades discussed above. The housings 16, 20 are then joined together so as to enclose therebetween the required electronic components, as described above. The electrodes and reservoirs are then placed between the sealed housings and the adhesive portion so as to complete the assembly of the device 100.

The present invention thus provides an electrotransport delivery device for delivering a therapeutic agent, for example, a drug such as fentanyl or sufentanil, through a body surface, e.g., skin, to achieve an analgesic effect. The drug salt is provided in a donor reservoir of an electrotransport delivery device as an aqueous salt solution. Most preferably, the aqueous solution is contained within a hydrophilic polymer matrix such as a hydrogel matrix. The drug salt is present in an amount sufficient to deliver the required doses transdermally by electrotransport over a delivery period of up to about 20 minutes, to achieve a systemic analgesic effect. The fentanyl/sufentanil salt typically comprises about 1 to 10 wt% of the donor reservoir formulation (including the weight of the polymeric matrix) on a fully hydrated basis, and more preferably about 1 to 5 wt% of the donor reservoir formulation on a fully hydrated basis. Although not critical to the device of the present invention, the applied electrotransport current density is typically in the range of about 50 to 150 µA/cm² and the applied electrotransport current is typically in the range of about 150 to 240 µA.

The anodic salt-containing hydrogel can suitably be made of any number of materials but preferably is composed of a hydrophilic polymeric material, preferably one that is polar in nature so as to enhance the drug stability. Suitable polar polymers for the hydrogel matrix comprise a variety of synthetic and naturally occurring polymeric materials. A preferred hydrogel formulation contains a suitable hydrophilic polymer, a buffer, a humectant, a thickener, water and a water soluble salt (e.g., HCl salt). A preferred hydrophilic polymer matrix is polyvinyl alcohol such as a washed and fully hydrolyzed polyvinyl alcohol (PVOH), e.g., Mowiol 66-100 commercially available from Hoechst Aktiengesellschaft. A suitable buffer is an ion exchange resin which is a copolymer of methacrylic acid and divinylbenzene in both an acid and salt form. One example of such a buffer is a mixture of Polacrilin (the copolymer of methacrylic acid and divinyl benzene available from Rohm & Haas, Philadelphia, PA) and the potassium salt thereof. A mixture of the acid and potassium salt forms of Polacrilin functions as a polymeric buffer to adjust the pH of the hydrogel. Use of a humectant in the hydrogel formulation is beneficial to inhibit the loss of moisture from the hydrogel. An example of a suitable humectant is guar gum. Thickeners are also beneficial in a hydrogel formulation. For example, a polyvinyl alcohol thickener such as hydroxypropyl methylcellulose (e.g., Methocel K100MP available from Dow Chemical, Midland, MI) aids in modifying the rheology of a hot polymer solution as it is dispensed into a mold or cavity. The hydroxypropyl methylcellulose increases in viscosity on cooling and significantly reduces the propensity of a cooled polymer solution to overfill the mold or cavity.

In one preferred embodiment, the anodic salt-containing hydrogel formulation comprises about 10 to 15 wt% polyvinyl alcohol, 0.1 to 0.4 wt% resin buffer, and about 1 to 2 wt% salt, preferably the hydrochloride salt. The remainder is water and ingredients such as humectants, thickeners, etc. The polyvinyl alcohol (PVOH)-based hydrogel formulation is prepared by mixing all materials, including the fentanyl or sufentanil salt, in a single vessel at elevated temperatures of about 90°C to 95°C for at least about 0.5 hr. The hot mix is then poured into foam molds and stored at freezing temperature of about -35°C overnight to cross-link the PVOH. Upon warming to ambient temperature, a tough elastomeric gel is obtained suitable for fentanyl electrotransport.

A suitable electrotransport device includes an anodic donor electrode, preferably comprised of silver, and a cathodic counter electrode, preferably comprised of silver chloride. The donor electrode is in electrical contact with the donor reservoir containing the aqueous solution of a salt. As described above, the donor reservoir is preferably a hydrogel formulation.
The counter reservoir also preferably comprises a hydrogel formulation containing a (e.g., aqueous) solution of a biocompatible electrolyte, such as, for example, citrate buffered saline.

In summary, the present invention provides an improved electrotransport device having an exterior housing made from an ethylene-octene copolymer, preferably an ENGAGE® copolymer. The use of ethylene-octene for at least the upper or exterior housing of the electrotransport device provides improved molding properties and an increased processing window when compared with the prior use of EVA-28, good flexibility and hardness in the final molded product, improved dimensional stability, a lower moisture vapor transmission rate which reduces the risk of corroding the electronic components, and no acetic acid off-gassing to corrode the electronic components or the mold and tooling during manufacturing of the housing.

While the present invention has been described with preferred embodiments, it is to be understood that variations and modifications may be resorted to as will be apparent to those skilled in the art. Such variations and modifications are to be considered within the purview and the scope of the claims appended hereto.

## Claims

1. An electrotransport device (100) for transporting an agent through a body surface by electrotransport, the device having a housing (16,20), said housing (16,20) enclosing electronic components (18,19,32,40), and at least one hydrated reservoir (26,28), wherein at least a portion of the housing (16,20) comprises an ethylene-octene copolymer.

2. The device of claim 1, wherein the ethylene-octene copolymer has an octene content of between 5.0 percent and 30.0 percent, preferably between 9.5 percent and 24 percent, more preferably approximately 20 percent.

3. The device of claim 1 or claim 2, wherein the ethylene-octene copolymer has a melt flow index between 0.5 and 35 dg/min, preferably of approximately 18 dg/min.

4. The device of any one of claims 1 to 3, wherein the ethylene-octene copolymer has a Shore A hardness value between 66 and 96, preferably of approximately 76.

5. The device of any one of claims 1 to 4, wherein the agent is a drug.

6. The device of claim 5, wherein the drug is a fentanyl or sufentanil salt analgesic drug.

7. The device of any one of claims 1 to 4, wherein the agent is a body analyte.

8. The device of any one of claims 1 to 7, wherein the device is adapted to be applied to skin, such as human skin.

9. The device of any one of claims 1 to 8, wherein the housing includes an upper housing portion (16) and a lower housing portion (20), the electronic components (18, 19,32,40) being enclosed between the upper and lower housing portions (16,20); and wherein at least said upper housing portion (16) is comprised of said ethylene-octene copolymer.

10. A method of manufacturing a transdermal electrotransport delivery or sampling device (100), the device including a housing (16,20) enclosing electronic components (18,19,32,40), the method comprising:
forming at least a portion of the housing (16,20) from an ethylene-octene copolymer; and
placing the electronic components (18,19,32,40) within the housing (16,20).

11. The method of claim 10, wherein said forming step includes injection molding at least a portion of the housing (16,20).

12. The method of claim 10, wherein said forming step includes thermoforming at least a portion of the housing (16,20).

13. The method of any one of claims 10 to 12, wherein said housing includes an upper housing portion (16) and a lower housing portion (20) and said forming step includes injection molding the upper housing portion (16) from the ethylene-octene copolymer.

14. The method of claim 13, wherein said forming step includes forming the lower housing portion (20) from the ethylene-octene copolymer.

15. The method of claim 10, which produces a device according to any one of claims 1 to 9.

## Patentansprüche

1. Elektrotransport-Vorrichtung (100) zum Transportieren eines Mittels durch eine Körperoberfläche mittels Elektrotransport, wobei die Vorrichtung ein Gehäuse (16, 20) aufweist, wobei das Gehäuse (16, 20) elektronische Komponenten (18, 19, 32, 40) und mindestens einen hydriertes Reservoir (26, 28) einschließt, worin mindestens ein Teil des Gehäuses (16, 20) ein Ethylen-Octen-Copolymer umfaßt.

2. Vorrichtung nach Anspruch 1, worin das Ethylen-Octen-Copolymer einen Octen-Gehalt von zwischen 5,0 % und 30,0 %, bevorzugterweise zwischen 9,5 % und 24 %, weiter bevorzugt von ungefähr 20 % hat.

3. Vorrichtung nach Anspruch 1 oder Anspruch 2, worin das Ethylen-Octen-Copolymer einen Schmelzindex zwischen 0,5 und 35 dg/min, bevorzugterweise von ungefähr 18 dg/min, hat.

4. Vorrichtung nach einem der Ansprüche 1-3, worin das Ethylen-Octen-Copolymer einen Shore A-Härtewert zwischen 66 und 96, bevorzugterweise von ungefähr 76, hat.

5. Anspruch nach einem der Ansprüche 1-4, worin das Mittel ein Wirkstoff ist.

6. Vorrichtung nach Anspruch 5, worin der Wirkstoff ein Fentanyl oder Sufentanil-Salz analgetischer Wirkstoff ist.

7. Anspruch nach einem der Ansprüche 1-4, worin das Mittel ein Körper-Analyt ist.

8. Vorrichtung nach einem der Ansprüche 1-7, worin die Vorrichtung geeignet ist, um auf Haut, wie z.B. humane Haut, appliziert zu werden.

9. Vorrichtung nach einem der Ansprüche 1-8, worin das Gehäuse einen oberen Gehäuseteil (16) und einen unteren Gehäuseteil (20) einschließt, die elektronischen Komponenten (18, 19, 32, 40) zwischen den oberen und unteren Gehäuseteilen (16, 20) eingeschlossen sind; und worin mindestens das obere Gehäuseteile (16) das Ethylen-Octen-Copolymer umfaßt.

10. Verfahren zur Herstellung einer transdermalen Elektrotransport-Vorrichtung oder Proben-Entnahmevorrichtung (100), wobei die Vorrichtung ein Gehäuse (16, 20) einschließlich elektronische Komponenten (18, 19, 32, 40) einschließt, wobei das Verfahren umfaßt:
Formen mindestens eines Teiles des Gehäuses (16, 20) aus einem Ethylen-Octen-Copolymer und
Platzieren der elektronischen Komponenten (18, 19, 32, 40) in das Gehäuse (16,20).

11. Verfahren nach Anspruch 10, worin der Formschritt Spritzgießen mindestens eines Teiles des Gehäuses (16, 20) einschließt.

12. Verfahren nach Anspruch 10, worin der Formschritt Thermoformen mindestens eines Teils des Gehäuses (16, 20) einschließt.

13. Verfahren nach einem der Ansprüche 10-12, worin das Gehäuse einen oberen Gehäuseteil (16) und einen unteren Gehäuseteil (20) einschließt, und der Formschritt ein Spritzgießen der oberen Gehäuseportion (16) aus dem Ethylen-Octen-Copolymer einschließt.

14. Verfahren nach Anspruch 13, worin der Formschritt ein Formen des unteren Gehäuseteils (20) aus dem Ethylen-Octen-Copolymer einschließt.

15. Verfahren nach Anspruch 10, das eine Vorrichtung gemäß einem der Ansprüche 1-9 ergibt.

## Revendications

1. Un dispositif d'électrotransport (100) pour transporter, par électrotransport, un agent à travers une surface corporelle, le dispositif comprenant un boîtier (16,20), ledit dispositif (16,20) renfermant des composants électroniques (18,19,32,40), et au moins un réservoir hydraté (26,28), dans lequel au moins une partie du boîtier (16,20) est composé d'un copolymère d'éthylène-octène.

2. Le dispositif de la revendication 1, dans lequel le copolymère d'éthylène-octène présente une teneur en octène d'entre 5,0 pour cent et 30,0 pour cent, de préférence d'entre 9,5 pour cent et 24 pour cent, de manière davantage préférée d'approximativement 20 pour cent.

3. Le dispositif de la revendication 1 ou de la revendication 2, dans lequel le copolymère d'éthylène-octène présente un indice de fusion entre 0,5 et 35 dg/min, de préférence d'approximativement 18 dg/min.

4. Le dispositif de l'une quelconque des revendications 1 à 3, dans lequel le copolymère d'éthylène-octène présente une valeur de dureté Shore A entre 66 et 96, de préférence d'approximativement 76.

5. Le dispositif de l'une quelconque des revendications 1 à 4, dans lequel l'agent est un médicament.

6. Le dispositif de la revendication 5, dans lequel le médicament est un médicament analgésique de sel de fentanyl ou de sufentanil.

7. Le dispositif de l'une quelconque des revendications 1 à 4, dans lequel l'agent est un analyte corporel.

8. Le dispositif de l'une quelconque des revendications 1 à 7, dans lequel le dispositif est adapté pour être appliqué à de la peau, telle qu'une peau humaine.

9. Le dispositif de l'une quelconque des revendications 1 à 8, dans lequel le boîtier comprend une partie de boîtier supérieure (16) et une partie de boîtier inférieure (20), les composants électroniques (18,19,32,40) étant enfermés entre les parties de boîtier supérieur et inférieure (16,20); et dans lequel au moins ladite partie de boîtier supérieure (16) est composée dudit copolymère d'éthylène-octène.

10. Un procédé de fabrication d'un dispositif de fourniture ou d'échantillonnage d'électrotransport transdermique (100), le dispositif comprenant un boîtier (16,20) renfermant des composants électroniques (18,19,32,40), le procédé comprenant les étapes consistant à :
former au moins une partie du boîtier (16,20) à partir d'un copolymère d'éthylène-octène; et
placer les composants électroniques (18,19,32,40) à l'intérieur du boîtier (16,20).

11. Le procédé de la revendication 10, dans lequel ladite étape de formation comprend un moulage par injection d'au moins une partie du boîtier (16,20).

12. Le procédé de la revendication 10, dans lequel ladite étape de formation comprend un thermoformage d'au moins une partie du boîtier (16,20).

13. Le procédé de l'une quelconque des revendications 10 à 12, dans lequel ledit boîtier comprend une partie de boîtier supérieure (16) et une partie de boîtier inférieure (20) et où ladite étape de formation comprend un moulage par injection de la partie de boîtier supérieure (16) à partir du copolymère d'éthylène-octène.

14. Le procédé de la revendication 13, dans lequel ladite étape de formation comprend la formation de la partie de boîtier inférieure (20) à partir du copolymère d'éthylène-octène.

15. Le procédé de la revendication 10, produisant un dispositif selon l'une quelconque des revendications 1 à 9.
